# EUROPEAN PATENT APPLICATION

(11) **EP 2 110 369 A1**
(43) Date of publication of application: **21.10.2009**
(21) Application number: 09003880.3
(22) Date of filing: 18.03.2009
(51) Int. Cl.: C07C 17/12, C07D 209/86, C09K 11/07, C07C 25/22

(54) **Method of synthesizing 9-aryl-10-iodoanthracene derivative and light-emitting material**

(30) Priority: 25.03.2008 JP 2008077893
(71) Applicant: SEMICONDUCTOR ENERGY LABORATORY CO., LTD., Atsugi-shi, Kanagawa-ken 243-0036 (JP)
(72) Inventor: Kawakami, Sachiko, Atsugi-shi Kanagawa-ken 243-0036 (JP)
(74) Representative: Grünecker, Kinkeldey, Stockmair & Schwanhäusser Anwaltssozietät

(57) **Abstract**

A method of efficiently synthesizing 9-aryl-10-iodoanthracene from 9-arylanthracene by a simple procedure is provided. By mixing an iodinating agent that is a substance having a structure of an amide group in which nitrogen of the amide group and iodine are directly bonded to each other, an acid, and 9-arylanthracene, iodine is introduced into the 10-position of 9-arylanthracene, whereby 9-aryl-10-iodoanthracene can be synthesized.

## Description

### BACKGROUND OF THE INVENTION

### 1. Field of the Invention

The present invention relates to a method of synthesizing a 9-aryl-10-iodoanthracene derivative and particularly to a method of iodinating 9-arylanthracene.

### 2. Description of the Related Art

Substances that can convert obtained energy into light (luminescent substances) are widely used in such fields as lighting, displays, medicine, and biology. Among these substances are not only inorganic compounds but also a variety of organic compounds, and utilization of such organic compounds has been promoted.

The wavelength of light emitted by a light-emitting substance is inherent in the substance. Anthracene skeletons are typical examples of skeletons exhibiting good blue light emission. Further, because anthracene skeletons have a carrier-transporting property, some of them are used for semiconductor layers of organic transistors or for carrier-transporting layers of organic EL elements.

As described above, a substance with an anthracene skeleton is very useful because of its wide band gap that enables even blue light emission and carrier-transporting property.

By an appropriate design of the kind or position of a substituent, a targeted property of an organic compound can be varied while a required property of the organic compound is maintained. For example, 9,10-diarylanthracene in which aryl groups are substituted at the 9-and 10-positions of anthracene exhibits blue light emission, and can improve stability as a substance, quantum yield of light emission, and uniformity of a film formed by evaporation while keeping a carrier-transporting property; therefore, 9,10-diarylanthracene having a variety of structures have been proposed (see Patent Document 1, for example).

As a method of introducing an aryl group into an anthracene skeleton, there is a method as disclosed in Patent Document 1, in which a position at which an aryl group is desirably introduced is halogenated by substitution of bromine or iodine at the position, and then coupling with aryl boronic acid, an aryl organoboron compound, or arylamine is conducted. In this case, in the synthesis of 9,10-diarylanthracene which is unsymmetrical where different substituents are substituted at the 9- and 10-positions, two different substituents are difficult to introduce at the same time. Usually, after 9-arylanthracene is first synthesized, another substituent is introduced at the 10-position.

As a method of halogenating the 10-position of 9-arylanthracene, a method called the Sandmeyer method as described in Non-Patent Document 1 is known.

[Patent Document 1] PCT International Publication No. 05/054162
[Non-Patent Document 1] Fredrik Norrsel and two others, Journal of Organic Chemistry, Vol.58, No. 18, pp. 4929-4932, 1993

### SUMMARY OF THE INVENTION

In the synthesis of a compound having a more complicated structure, an aryl group is coupled with an anthracene skeleton, and then further coupling is sometimes repeated in order to introduce a substituent into the introduced aryl group. In such a case, it is preferable that a halogen group be preliminarily introduced into arylboronic acid (alternatively, an aryl organoboron compound or arylamine) that is first coupled with an anthracene skeleton.

When an anthracene skeleton is coupled with arylboronic acid or the like into which a halogen group is preliminarily introduced, in order that coupling of the arylboronic acid or the like and the anthracene skeleton may preferentially proceed while inhibiting a coupling reaction between the arylboronic acids into each of which the halogen group is preliminarily introduced, the halogen group that is introduced into the anthracene skeleton is preferably an iodo group containing iodine. This is because it is likely that boronic acid (alternatively, organoboron or an amino group) in arylboronic acid (alternatively, an aryl organoboron compound or arylamine), which reacts with a halogen group in a coupling reaction, will preferentially react with an iodo group instead of with any other halogen group (e.g., a bromo group or a chloro group).

As methods of iodinating the 10-position of 9-arylanthracene, the aforementioned Sandmeyer method, a method in which bromination is performed and then bromine is substituted with iodine, and the like are known.

However, the Sandmeyer method described in Non-Patent Document 1 and a method in which bromination is performed and then bromine is substituted with iodine are complicated and very inefficient. This is because each method involves introduction of a substituent that is different from iodine into a position of 9-arylanthracene, which is desirably iodinated, and therefore needs to increase the number of reaction steps, to prepare a reagent for a reaction for each step, and to repeat a procedure in which a substance is collected and purified. In other words, synthesis of 9-aryl-10-iodoanthracene from 9-arylanthracene by one reaction can be expected to improve yield and to reduce time and material costs, and thus has industrial advantages over the synthesis by two steps. Further, in Patent Document 1, although iodination of the 10-position of 9-arylanthracene is described, an iodination method itself is not described.

Therefore, in the present invention, a method of efficiently synthesizing 9-aryl-10-iodoanthracene by a simple procedure is provided.

The present inventor has found that, by use of an iodinating agent that is a substance having a structure of an amide group in which nitrogen of the amide group and iodine are directly bonded to each other (i.e., an iodinating agent that has an amide bond in which iodine is directly bonded to nitrogen of the amide bond) and an acid together, the 10-position of 9-arylanthracene can be rapidly and directly iodinated in high yield.

An example of the present invention is a method of synthesizing 9-aryl-10-iodoanthracene. In the method, iodine is introduced into the 10-position of 9-arylanthracene by mixing an iodinating agent that is a substance having a structure of an amide group in which nitrogen of the amide group and iodine are directly bonded to each other, an acid, and 9-arylanthracene.

Further, an example of the present invention is a method of synthesizing 9-aryl-10-iodoanthracene. In the method, iodine is introduced into the 10-position of 9-arylanthracene by mixing an iodinating agent having an amide bond in which iodine is directly bonded to nitrogen of the amide bond, an acid, and 9-arylanthracene.

By using a method as described above, the 10-position of 9-arylanthracene can be directly iodinated by stirring at room temperature or with slight heating, which is a simple procedure and a mild condition. Such a method, in which 9-aryl-10-iodoanthracene can be synthesized from 9-arylanthracene by one step, is very efficient.

Moreover, in a method according to the present invention, a solvent may be further used for the synthesis. When the acid that is used is liquid, the acid may also serve as a solvent.

As the acid, a substance serving as an acid for the iodinating agent (a substance having a structure of an amide group in which nitrogen of the amide group and iodine are directly bonded to each other, or a substance having an amide bond in which iodine is directly bonded to nitrogen of the amide bond) is used. Although an acid meeting any definition, such as a Lewis acid or a Bronsted acid, may be used, a Bronsted acid is preferably used. An indication of a Bronsted acid is a substance with the properties of Arrhenius acid, which shows acidity when the substance is dissolved in water. Specifically, acetic acid, sulfuric acid, trifluoroacetic acid, or the like is preferable. Alternatively, in the case of using a Lewis acid, aluminum(III) chloride or the like is used.

Note that the amide structure (bond) in the iodinating agent may be a part included in an imide structure (bond). In this case, it can be said that the iodinating agent is a substance in which iodine is directly bonded to nitrogen forming the imide structure (bond). Further, the amide structure in the iodinating agent may be a diacylamide structure.

By a synthesis method according to the present invention, 9-aryl-10-iodoanthracene can be efficiently synthesized. Further, a 9,10-diarylanthracene derivative formed using 9-aryl-10-iodoanthracene as a material can be simply synthesized at a low cost.

### BRIEF DESCRIPTION OF THE DRAWINGS

FIGS. 1A and 1B show ¹H NMR charts of 9-iodo-10-phenylanthracene synthesized in Synthesis Example 1.
FIG 2 shows an absorption spectrum and an emission spectrum of a toluene solution of PCBAPA.
FIG 3 shows an absorption spectrum and an emission spectrum of a thin film of PCBAPA.
FIG 4 shows an absorption spectrum and an emission spectrum of a toluene solution of PCCPA.
FIG 5 shows an absorption spectrum and an emission spectrum of a thin film of PCCPA.
FIG 6 schematically illustrates a structure of a light-emitting element.
FIG. 7 shows the current density vs. luminance characteristic of a light-emitting element fabricated in Example 5.
FIG 8 shows the voltage vs. luminance characteristic of the light-emitting element fabricated in Example 5.
FIG 9 shows the luminance vs. current efficiency characteristic of the light-emitting element fabricated in Example 5.
FIG. 10 shows the luminance vs. external quantum efficiency of the light-emitting element fabricated in Example 5.
FIG. 11 shows an emission spectrum of the light-emitting element fabricated in Example 5.
FIG. 12 shows a luminance degradation curve of the light-emitting element fabricated in Example 5.

### DETAILED DESCRIPTION OF THE INVENTION

Hereinafter, an embodiment of the present invention will be described. Note that the present invention can be implemented in many different modes, and it is easily understood by those skilled in the art that a variety of changes and modifications can be made without departing from the spirit and scope of the present invention. Therefore, the present invention should not be interpreted as being limited to the described content of the embodiment described below.

### (Embodiment 1)

In the present invention, as illustrated in a synthesis scheme (a) below, by mixing 9-arylanthracene represented by a general formula (M1), an iodinating agent, and an acid, 9-aryl-10-iodoanthracene represented by a general formula (G1) can be rapidly obtained with high efficiency.

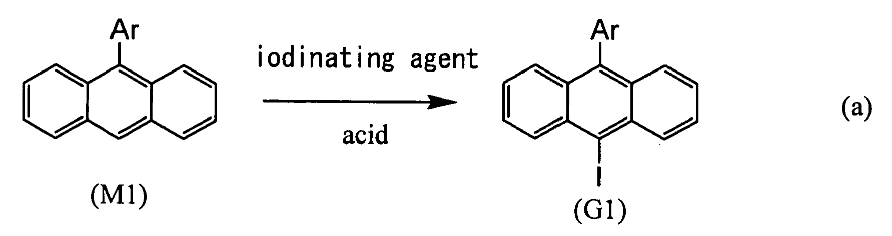

In the formula, Ar represents an aryl group that has 6 to 13 carbon atoms in a ring. Specifically, there are a phenyl group, a naphthyl group, a biphenyl group, a fluorenyl group, and the like. The aryl group may have a substituent; in this case, as the substituent, there are an alkyl group having 1 to 4 carbon atoms, a halogen group, a haloalkyl group, an aryl group having 6 to 10 carbon atoms, and the like.

The reaction proceeds by only stirring at room temperature, and thus 9-aryl-10-iodoanthracene can be efficiently synthesized from 9-arylanthracene by a very simple procedure. Note that slight heating is preferable.

Further, since the synthesis is completed in only one step, a procedure in which a substance that is the object is collected and purified needs performing only one time. Thus, 9-aryl-10-iodoanthracene can be efficiently synthesized.

As the iodination agent, a substance having a structure of an amide group in which nitrogen of the amide group and iodine are directly bonded to each other, or a substance having an amide bond in which iodine is directly bonded to nitrogen of the amide bond is used. In the iodinating agent, nitrogen may be further bonded to carbon of the amide group, and the nitrogen and iodine may be directly bonded to each other. Further, the equivalent of the iodinating agent depends on the number of iodine atoms bonded to a nitrogen atom.

Examples of the iodinating agent include compounds represented by general formulae (1) to (3) below. In the formulae, R¹ represents any one of iodine, an alkyl group, an aryl group, or hydrogen, R² and R³ independently represent any one of an alkyl group, an aryl group, or the like and may be directly bonded to each other to form a ring structure, R⁴ and R⁵ independently represent any one of an alkyl group, an aryl group, or the like and may be directly bonded to each other to form a ring structure, R⁶ and R⁷ independently represent any one of an alkyl group, an aryl group, or the like and may be directly bonded to each other to form a ring structure, and R⁸ represents any one of iodine, an alkyl group, aryl, or hydrogen.

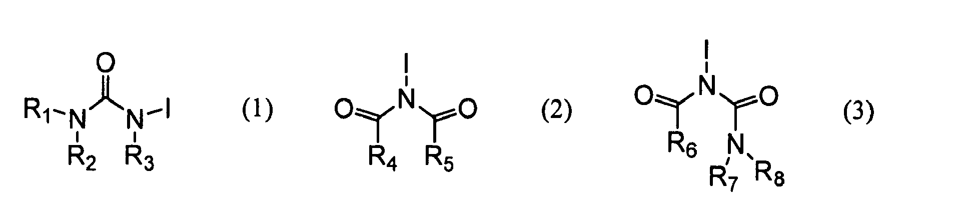

Further, the structures in the general formulae (1), (2), and (3), in each of which nitrogen of the amide group and iodine are directly bonded to each other, refer to, for example, the structure of the portion enclosed by dotted lines in a general formula (1) below, the structure of the portion enclosed by dotted lines in a general formula (2) below, and the structure of the portion enclosed by dotted lines in a general formula (3) below, respectively.

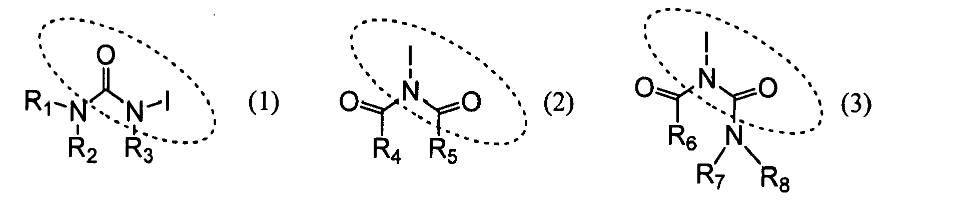

As specific examples of the iodinating agent, there are 1,3-diiodo-5,5-dimethylimidazolidine-2,4-dione (abbreviation: DIH) represented by a structural formula (4) below, *N*-iodosuccinimide (abbreviation: NIS) represented by a structural formula (5) below, 2,4,6,8-tetraiodo-2,4,6,8-tetraazabicyclo[3,3,0]octane-3,7-dione represented by a structural formula (6), 2-iodo-2,4,6,8-tetraazabicyclo[3,3,0]octane-3-,7-dione represented by a structural formula (7), and the like. Naturally, the iodinating agent is not limited to these four examples.

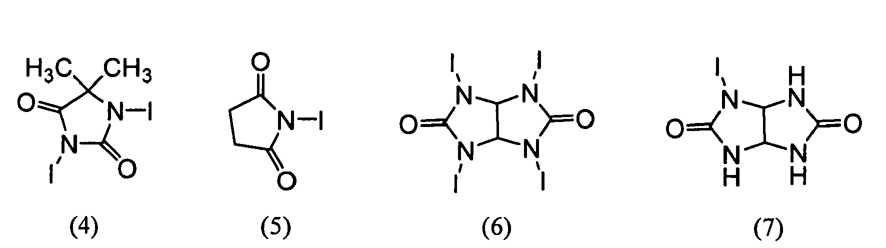

Further, the structures having an amide group in which nitrogen of the amide group and iodine are directly bonded to each other in these iodinating agents are like the structures of the portions enclosed by dotted lines in structural formulae (8) to (11) below.

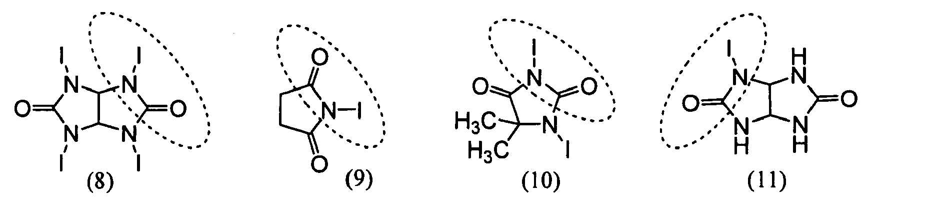

There is no limitation on the amount of the iodinating agent. Based on the number of iodine atoms, the number of the equivalents is preferably in the range of 1 to 5, more preferably in the range of 1 to 2 in terms of yield.

Among examples of the iodinating agent, a compound having a diacylamide structure (also referred to as an imide structure) as represented by a structural formula (12) below is preferable because of its low cost. The term diacylamide structure (imide structure) means the structure of a portion enclosed by dotted lines in the structure represented by the structural formula (12). Note that in the formula, R¹ represents any one of iodine, an alkyl group, an aryl group, or hydrogen, R² and R³ independently represent any one of an alkyl group, an aryl group, or the like and may be directly bonded to each other to form a ring structure, and R⁴ and R⁵ independently represent any one of an alkyl group, an aryl group, or the like and may be directly bonded to each other to form a ring structure.

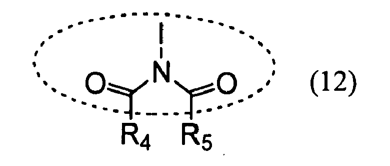

As the acid, a substance serving as an acid for the above iodinating agent (a substance having a structure of an amide group in which nitrogen of the amide group and iodine are directly bonded to each other, or a substance having an amide bond in which iodine is directly bonded to nitrogen of the amide bond) is used. Although an acid meeting any definition, such as a Lewis acid or a Bronsted acid, may be used, a Bronsted acid is preferably used. An indication of a Bronsted acid is a substance with the properties of Arrhenius acid, which shows acidity when dissolved in water. Examples of the acid include Lewis acids such as boron trifluoride and aluminum(III) chloride, Bronsted acids such as acetic acid, sulfuric acid, and trifluoroacetic acid. Use of a liquid acid is preferable since the acid can also be used as a solvent.

Note that in the synthesis without using an acid by the synthesis method of Embodiment 1, it takes an enormous amount of time to iodinate the 10-position of 9-arylanthracene and only a small amount of object is obtained. Alternatively, with the use of a simple substance of iodine instead of the above iodinating agent, the 10-position of 9-arylanthracene is difficult to iodinate. In other words, only by making the above iodinating agent and an acid act together on 9-arylanthracene, the 10-position of 9-arylanthracene can be iodinated directly, rapidly, and simply. This is because an iodination reaction proceeds due to a substance for iodination which is produced by a reaction of iodine in the above iodinating agent and the acid. A production example of a substance for iodination which enables such a reaction to proceed is illustrated in a reaction formula (d) below. A compound (P1) produced in the reaction formula (d) is an example of the substance for iodination which is produced by the reaction of iodine in the above iodinating agent and the acid.

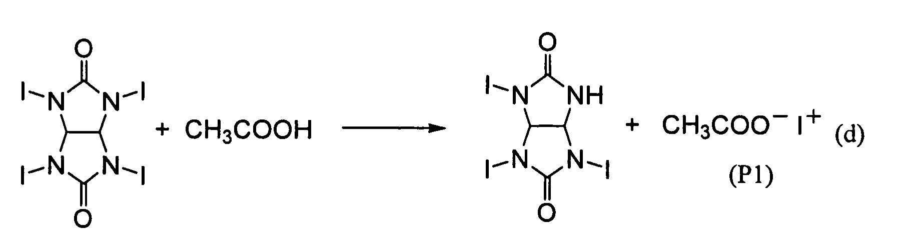

In the synthesis method of Embodiment 1, a solvent may be used. In this case, there is no limitation on the solvent that is used, and the following substances can be used alone or in combination: aromatic hydrocarbons such as benzene, toluene, and xylene; ethers such as 1,2-dimethoxyethane, diethyl ether, methyl-*t*-butyl ether, tetrahydrofuran, and dioxane; saturated hydrocarbons such as pentane, hexane, heptane, octane, and cyclohexane; halogens such as dichloromethane, chloroform, carbon tetrachloride, 1,2-dichloroethane, and 1,1,1-trichloroethane; nitriles such as acetonitrile and benzonitrile; esters such as ethyl acetate, methyl acetate, and butyl acetate; acetic acid (glacial acetic acid); water; and the like. When water is used, it is preferably mixed with an organic solvent.

As described above, 9-aryl-10-iodoanthracene can be efficiently synthesized from 9-arylanthracene by a simple procedure, according to the present invention.

Further, in the synthesis method of Embodiment 1, without any special procedure, the object can be obtained under mild conditions where materials are prepared and only stirred at room temperature (or stirred with slight heating). Thus, it can be said that the synthesis method described in Embodiment 1 is very suitable for automated large-scale production. Accordingly, 9-aryl-10-iodoanthracene and an anthracene derivative which is formed using 9-aryl-10-iodoanthracene as a material can be obtained at a low cost.

Further, 9-aryl-10-iodoanthracene which is synthesized by a method as described above is coupled with aryl boronic acid or an aryl organoboron compound by using a palladium catalyst, a nickel catalyst, or the like, or coupled with arylamine by using a palladium catalyst, a copper catalyst, or the like, whereby 9, 1 0-diaryl anthracene in which aryl groups are bonded to the 9- and 10-positions of anthracene can be obtained.

Note that by further repeating coupling, 9,10-diarylanthracene having a more complicated structure can also be synthesized. In this case, a substance in which a halogen group such as bromine or chlorine is preliminarily introduced into aryl boronic acid, an aryl organoboron compound, or arylamine, which is to be coupled with 9-aryl-10-iodoanthracene, is preferably used.

### [Example 1]

In Example 1, the results of synthesis are described in which 1,3-diiodo-5,5-dimethylimidazolidine-2,4-dione (abbreviation: DIH) and acetic acid were used as the iodinating agent and the acid, respectively, and accordingly 9-iodo-10-phenylanthracene was synthesized from 9-phenylanthracene.

### «Synthesis Example 1: One Equivalent of Iodinating Agent»

In a 100 mL Mayer flask was put 500 mg (2.0 mmol) of 9-phenylanthracene. To the flask was added 40 mL of acetic acid, followed by heating at about 70 °C, and 9-phenylanthracene was dissolved therein. To this solution was added 370 mg (1.0 mmol) of 1,3-diiodo-5,5-dimethylimidazolidine-2,4-dione (abbreviation: DIH) as the iodinating agent. This solution was stirred in air at 70 °C for 5 hours. After the solution was stirred, to this solution were added about 50 mL of water and about 50 mL of chloroform. This mixture was washed with water twice, and the aqueous layer was extracted with chloroform. The extract solution was combined with the organic layer and washed with a saturated saline solution, and then the organic layer was dried with magnesium sulfate. This mixture was gravity filtered, and the obtained filtrate was concentrated to give a brown solid. The obtained solid was purified by high-performance liquid chromatography (HPLC) (mobile phase: chloroform) to give 405 mg of a yellow solid at a yield of 54 %. The synthesis scheme is illustrated in (a-1).

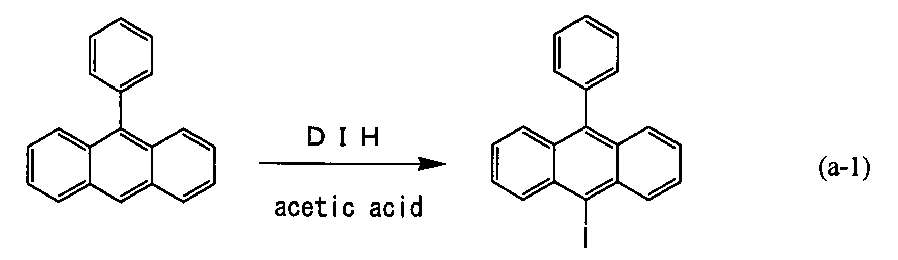

The obtained solid was analyzed by ¹H NMR. The analysis data are shown as follows:
¹H NMR (CDCl₃, 300MHz): δ = 7.33-7.42 (m, 4H), 7.52-7.61 (m, 7H), 8.56 (d, J=9.0Hz,2H)

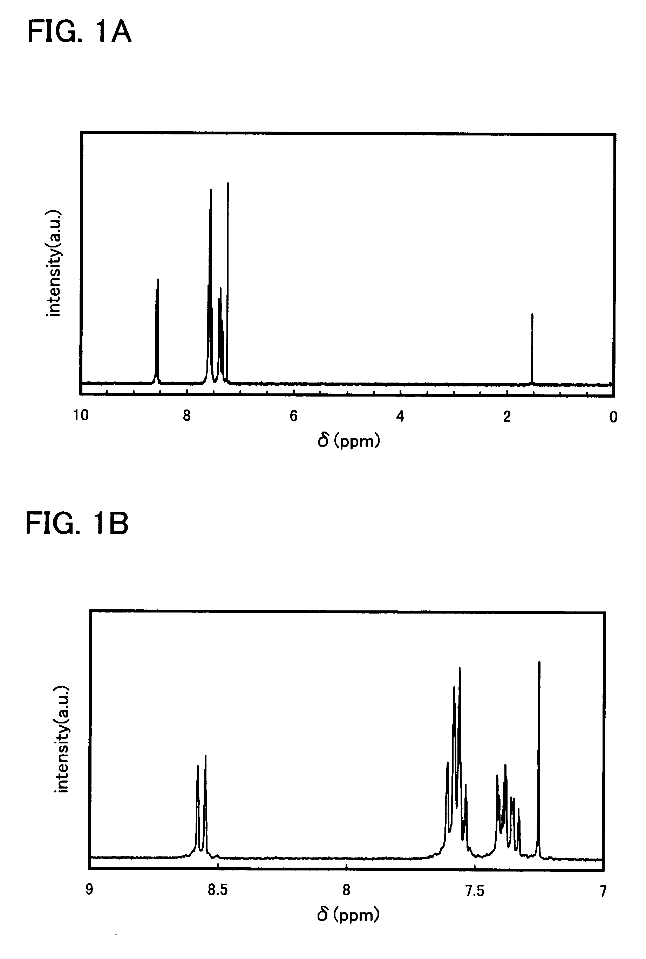

Further, FIGS. 1A and 1B show the ¹H NMR charts. FIG. 1B is a chart in which the range of 7.0 to 9.0 ppm in FIG. 1A is enlarged.

### «Synthesis Example 2: Two Equivalents of Iodinating Agent»

In a 100 mL Mayer flask was put 500 mg (2.0 mmol) of 9-phenylanthracene. To the flask was added 40 mL of acetic acid, followed by heating at about 70 °C, and 9-phenylanthracene was dissolved therein. To this solution was added 746 mg (2.0 mmol) of 1,3-diiodo-5,5-dimethylimidazolidine-2,4-dione (abbreviation: DIH), and this solution was stirred in air at room temperature for 17 hours. After the solution was stirred, to this solution was added about 50 mL of water, whereby a solid was precipitated. This solid was collected by suction filtration and then dissolved in toluene. This solution was filtered through alumina, Celite (a product of Wako Pure Chemical Industries, Ltd., Catalog No. 531-16855), and Florisil (a product of Wako Pure Chemical Industries, Ltd., Catalog No. 540-00135), and the obtained filtrate was concentrated to give the object of the synthesis as 350 mg of a light yellow solid at a yield of 47%. The reaction scheme is similar to that of the above (a-1).

### «Synthesis Example 3: Four Equivalents of Iodinating Agent»

In a 100 mL Mayer flask was put 500 mg (2.0 mmol) of 9-phenylanthracene. To the flask was added 40 mL of acetic acid, followed by heating at about 70 °C, and 9-phenylanthracene was dissolved therein. To this solution was added 1.5 g (3.9 mmol) of 1,3-diiodo-5,5-dimethylimidazolidine-2,4-dione (abbreviation: DIH). This solution was stirred in air at 70 °C for 5 hours. After the solution was stirred, to this solution were added about 50 mL of water and about 50 mL of chloroform. This mixture was washed with water twice, and the aqueous layer was extracted with chloroform. The extract solution was combined with the organic layer and washed with a saturated saline solution, and then the organic layer was dried with magnesium sulfate. This mixture was gravity filtered, and the obtained filtrate was concentrated to give the object of the synthesis as 218 mg of a brown solid a yield of 37 %. The reaction scheme is similar to that of the above (a-1).

### «Synthesis Example 4: 1.5 Equivalents of Iodinating Agent, Scale-up»

The results of a scale-up iodination reaction of 9-phenylanthracene with the use of DIH and acetic acid are described.

In a 500 mL Mayer flask was put 4.5 g (18 mmol) of 9-phenylanthracene. To the flask was added 200 mL of acetic acid, followed by heating at 70 °C, and 9-phenylanthracene was dissolved therein. To this solution was added 5.2 g (13 mmol) of 1,3-diiodo-5,5-dimethylimidazolidine-2,4-dione (abbreviation: DIH). This solution was stirred in air at 70 °C for 3 hours. After the solution was stirred, to this solution were added about 100 mL of water and about 200 mL of chloroform. This mixture was washed with water twice, and the aqueous layer was extracted with chloroform. The extract solution was combined with the organic layer and washed with a saturated saline solution, and then the organic layer was dried with magnesium sulfate. This mixture was gravity filtered, and the obtained filtrate was concentrated to give a brown solid. This solid was washed with hexane to give the object of the synthesis as 5.8 g of a yellow solid at a yield of 86 %. The reaction scheme is similar to that of the above (a-1).

Table 1 shows the results of Synthesis Examples 1 to 4.

**Table 1**

| | Equivalents of DIH | reaction condition | yield(%) |
|---|---|---|---|
| Synthesis Example 1 | 1 | 70°C,5hours | 54 |
| Synthesis Example 2 | 2 | room temperature,17hours | 47 |
| Synthesis Example 3 | 4 | 70°C,5hours | 37 |
| Synthesis Example 4 | 1.5 | 70°C,3hours | 86 |

As described above, by using a synthesis method according to the present invention, 9-iodo-10-phenylanthracene was able to be efficiently synthesized from 9-phenylanthracene by a simple procedure in one step. Further, a scale-up of the reaction from milligram to gram significantly improved the yield. This is thought to be owing to a reduction in loss during collection and purification after reaction. In other words, monoiodo body, which was the object of the synthesis, was produced as the principal product while generation of a by-product was suppressed; therefore, an iodination reaction according to the present invention can be said to have great potential in the industry. Furthermore, since a scale-up as in Synthesis Example 4 enabled the object to be obtained at a yield of 86 %, which was very high, 9-aryl-10-iodoanthracene or an organic compound formed using 9-aryl-10-iodoanthracene as a material can be synthesized in an industrially advantageous manner by using an iodination method according to the present invention.

### [Example 2]

In Example 2, the results of synthesis are described in which *N*-iodosuccinimide (abbreviation: NIS) and acetic acid were used as the iodinating agent and the acid, respectively, and accordingly 9-iodo-10-phenylanthracene was synthesized from 9-phenylanthracene.

### «Synthesis Example 5: One Equivalent of Iodinating Agent»

In a 100 mL Mayer flask was put 500 mg (2.0 mmol) of 9-phenylanthracene. To the flask was added 40 mL of acetic acid, followed by heating at about 70 °C, and 9-phenylanthracene was dissolved therein. To this solution was added 450 mg (2.0 mmol) of *N*-iodosuccinimide (abbreviation: NIS) as the iodinating agent. This solution was stirred in air at 70 °C for 5 hours. After the solution was stirred, to this solution were added about 50 mL of water and about 50 mL of chloroform. This mixture was washed with water twice, and the aqueous layer was extracted with chloroform. The extract solution was combined with the organic layer and washed with a saturated saline solution, and then the organic layer was dried with magnesium sulfate. This mixture was gravity filtered, and the obtained filtrate was concentrated to give a brown solid. The obtained brown solid was purified by high-performance liquid chromatography (HPLC) (mobile phase: chloroform) to give the object of the synthesis as 378 mg of a yellow solid at a yield of 50 %. The synthesis scheme is illustrated in (a-2).

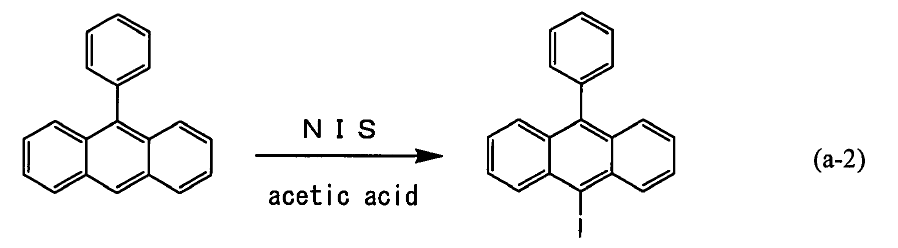

### «Synthesis Example 6: Two Equivalents of Iodinating Agent»

In a 100 mL Mayer flask was put 500 mg (2.0 mmol) of 9-phenylanthracene. To the flask was added 40 mL of acetic acid, followed by heating at about 70 °C, and 9-phenylanthracene was dissolved therein. To this solution was added 900 mg (4.0 mmol) of *N*-iodosuccinimide (abbreviation: NIS), and this solution was stirred in air at room temperature for 17 hours. After the solution was stirred, to this solution was added about 50 mL of water, whereby a solid was precipitated. This solid was collected by suction filtration, and the obtained solid was dissolved in toluene. This solution was filtered through alumina, Celite, and Florisil, and the obtained filtrate was concentrated to give the object of the synthesis as 350 mg of a light yellow solid at a yield of 47 %. The reaction scheme is similar to that of the above (a-2).

Table 2 shows the results of Synthesis Examples 5 and 6.

**Table 2**

| | Equivalents of NIS | reaction condition | yield(%) |
|---|---|---|---|
| Synthesis Example 5 | 1 | 70°C,5hours | 50 |
| Synthesis Example 6 | 2 | room temperature,17hours | 47 |

As described above, by using a synthesis method according to the present invention, 9-aryl-10-iodoanthracene was able to be efficiently synthesized from 9-arylanthracene by a simple procedure in one step.

### (Comparative Example)

Although a reaction similar to those of the above Synthesis Examples 1 to 6 was performed using ethyl acetate instead of acetic acid at room temperature for 17 hours, 9-iodo-10-phenylanthracene was not able to be synthesized. Further, although an attempt to iodinate 9-phenylanthracene was made under conditions where using iodine, orthoperiodic acid, acetic acid, and acetic anhydride are used, 9-iodo-10-phenylanthracene was not able to be obtained.

### [Example 3]

In Example 3, an example is described in which a 9,10-diarylanthracene derivative is synthesized by coupling of 9-iodo-10-phenylanthracene synthesized in Example 1 or Example 2 and aromatic hydrocarbon having a halogen group.

### «Synthesis Example 7: Synthesis of 4-(10-Phenyl-9-anthryl)-4'-(9-phenyl-9H-carbazol-3-yl)triphenylamine (abbreviation: PCBAPA)»

In Synthesis Example 7, a method of synthesizing 4-(10-phenyl-9-anthryl)-4'-(9-phenyl-9*H*-carbazol-3-yl)triphenylamine (abbreviation: PCBAPA) represented by a structural formula (13) below is specifically described.

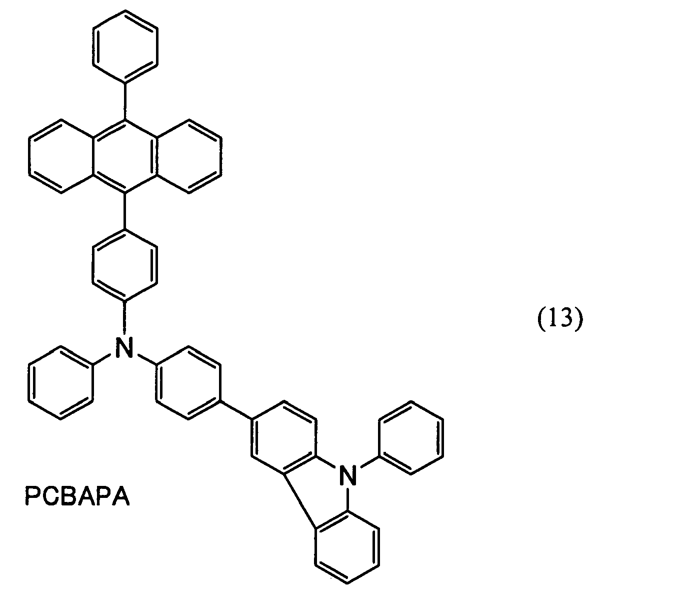

First, a method is specifically described in which 9-(4-bromophenyl)-10-phenylanthracene is synthesized by coupling of 9-phenyl-10-iodoanthracene and *p*-bromophenyl boronic acid which is aromatic hydrocarbon to which bromine which is halogen is bonded.

### [Step 1: Synthesis of 9-(4-Bromophenyl)-10-phenylanthracene]

A method of synthesizing 9-(4-bromophenyl)-10-phenylanthracene is described in which 9-iodo-10-phenylanthracene as synthesized in Example 1 or Example 2 is used as a starting material. In a 50mL three-necked flask were put 1.0 g (2.63 mmol) of 9-iodo-10-phenylanthracene, 542 mg (2.70 mmol) of *p*-bromophenyl boronic acid, 46 mg (0.03 mmol) of tetrakis(triphenylphosphine)palladium(0), 3 mL of an aqueous potassium carbonate solution (2 mol/l), and 10 mL of toluene. This mixture was stirred under nitrogen stream at 80 °C for 9 hours. After the mixture was stirred, to this mixture was added about 20 mL of toluene. Then, the mixture was suction-filtered through Florisil (a product of Wako Pure Chemical Industries, Ltd., Catalog No. 540-00135), Celite (a product of Wako Pure Chemical Industries, Ltd., Catalog No. 531-16855), and alumina. The obtained filtrate was washed with water and a saturated saline solution, followed by drying with magnesium sulfate. This mixture was gravity filtered, and the obtained filtrate was concentrated to give a solid. This solid was recrystallized with chloroform/hexane, whereby 9-(4-bromophenyl)-10-phenylanthracene was obtained as 562 mg of a light brown solid at a yield of 45 %. The synthesis scheme of Step 1 is illustrated in (b-1) below.

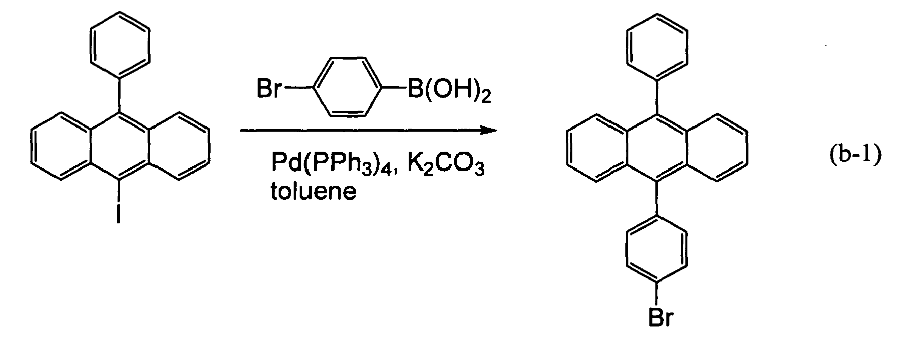

Next, a method of synthesizing 4-(9-phenyl-9*H-*carbazol-3-yl)diphenylamine (abbreviation: PCBA), which is to be coupled with 9-(4-bromophenyl)-10-phenylanthracene in order to synthesize the object, PCBAPA, is described in Steps 2 and 3.

### [Step 2: Synthesis of 9-Phenyl-9H-carbazole-3 -boronic acid]

In a 500 mL three-necked flask was put 10 g (31 mmol) of 3-bromo-9-phenyl-9*H-*carbazole. The atmosphere in the flask was replaced with nitrogen. To the flask was added 150 mL of tetrahydrofuran (THF), and 3-bromo-9-phenyl-9*H*-carbazole was dissolved therein. This solution was cooled to -80 °C. To this solution was added 20 mL (32 mmol) of n-butyllithium (a 1.58 mol/L hexane solution) by being dropped with a syringe. After completion of the dropping, the solution was stirred at the same temperature for one hour. After the solution was stirred, to this solution was added 3.8 mL (34 mmol) of trimethyl borate, and the mixture was stirred for about 15 hours while the temperature of the mixture was being brought back to room temperature. After the solution was stirred, to this solution was added about 150 mL (1.0 mol/L) of dilute hydrochloric acid, followed by stirring for one hour. Then, the aqueous layer of this mixture was extracted with ethyl acetate. The extract solution and the organic layer were combined, and the mixture was washed with saturated sodium hydrogen carbonate. The organic layer was dried with magnesium sulfate, and then the mixture was gravity filtered. The obtained filtrate was concentrated to give a light-brown oily substance. This oily substance was dried under reduced pressure to give the object of the synthesis as 7.5 g of a light brown solid at a yield of 86 %. The synthesis scheme of Step 2 is illustrated in (b-2) below.

,

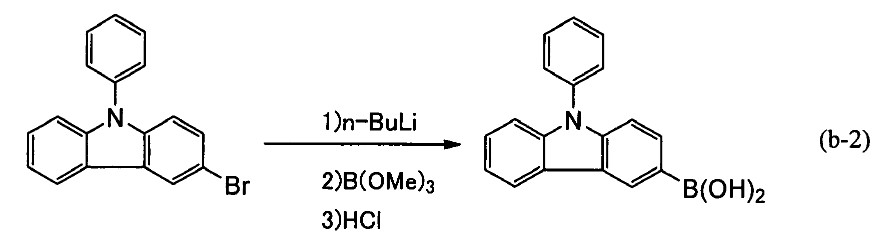

### [Step 3: Synthesis of 4-(9-Phenyl-9H-carbazol-3-yl)diphenylamine (abbreviation: PCBA)]

In a 500 mL three-necked flask were put 6.5 g (26 mmol) of 4-bromodiphenylamine, 7.5 g (26 mmol) of 9-phenyl-*H*-carbazole-3-boronic acid, and 400 mg (1.3 mmol) of tri(*o*-tolyl)phosphine. The atmosphere in the flask was replaced with nitrogen. To this mixture were added 100 mL of toluene, 50 mL of ethanol, and 14 mL of an aqueous potassium carbonate solution (2 mol/l). This mixture was degassed while being stirred under reduced pressure. After the mixture was degassed, to this mixture was added 67 mg (30 mmol) of palladium(II) acetate. This mixture was refluxed under nitrogen stream at 100 °C for 10 hours. After the mixture was refluxed, the aqueous layer of this mixture was extracted with toluene. The extract solution and the organic layer were combined, and the mixture was washed with a saturated saline solution. The organic layer was dried with magnesium sulfate. Then, the mixture was gravity filtered. The obtained filtrate was concentrated to give a light-brown oily substance. This oily substance was purified by silica gel column chromatography (developing solvent, hexane:toluene = 4:6). A white solid obtained by the purification was recrystallized with dichloromethane/hexane to give PCBA as 4.9 g of a white solid at a yield of 45 %. The synthesis scheme of Step 3 is illustrated in (b-3) below.

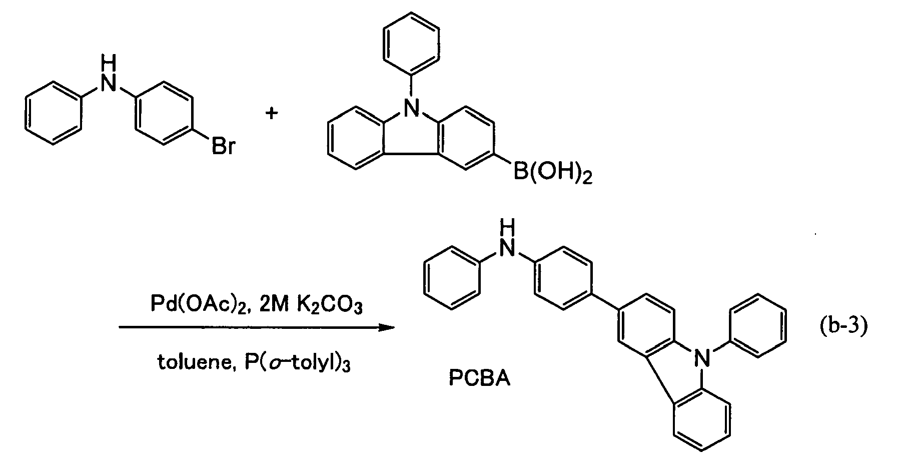

Lastly, a method of synthesizing PCBAPA by coupling of 9-(4-bromophenyl)-10-phenylanthracene and PCBA is described.

### [Step 4: Synthesis of PCBAPA]

In a 300 mL three-necked flask were put 7.8 g (12 mmol) of 9-(4-bromophenyl)-10-phenylanthracene as synthesized in Step 1, 4.8 g (12 mmol) of PCBA as synthesized in Steps 2 and 3, and 5.2 g (52 mmol) of sodium *tert*-butoxide. The atmosphere in the flask was replaced with nitrogen. To this mixture were added 60 mL of toluene and 0.30 mL of tri(tert-butyl)phosphine (a 10 wt% hexane solution). This mixture was degassed while being stirred under reduced pressure. After the mixture was degassed, to this mixture was added 136 mg (0.24 mmol) of bis(dibenzylideneacetone)palladium(0). This mixture was stirred at 100 °C for 3 hours. After the mixture was stirred, to this mixture was added about 50 mL of toluene, and the mixture was suction-filtered through Celite (a product of Wako Pure Chemical Industries, Ltd., Catalog No. 531-16855), alumina, and Florisil (a product of Wako Pure Chemical Industries, Ltd., Catalog No. 540-00135). The obtained filtrate was concentrated to give a yellow solid. This solid was recrystallized with toluene/hexane to give PCBAPA, which was the object of the synthesis, as 6.6 g of a light yellow solid at a yield of 75 %. Then, 3.0 g of the obtained light yellow solid was sublimated and purified by train sublimation. For sublimation purification conditions, PCBAPA was heated at 350 °C under a pressure of 8.7 Pa with argon gas at a flow rate of 3.0 mL/min. After the sublimation purification, PCBAPA was obtained as 2.7 g of a light yellow solid at a yield of 90 %. In addition, the synthesis scheme of Step 4 is illustrated in (b-4) below.

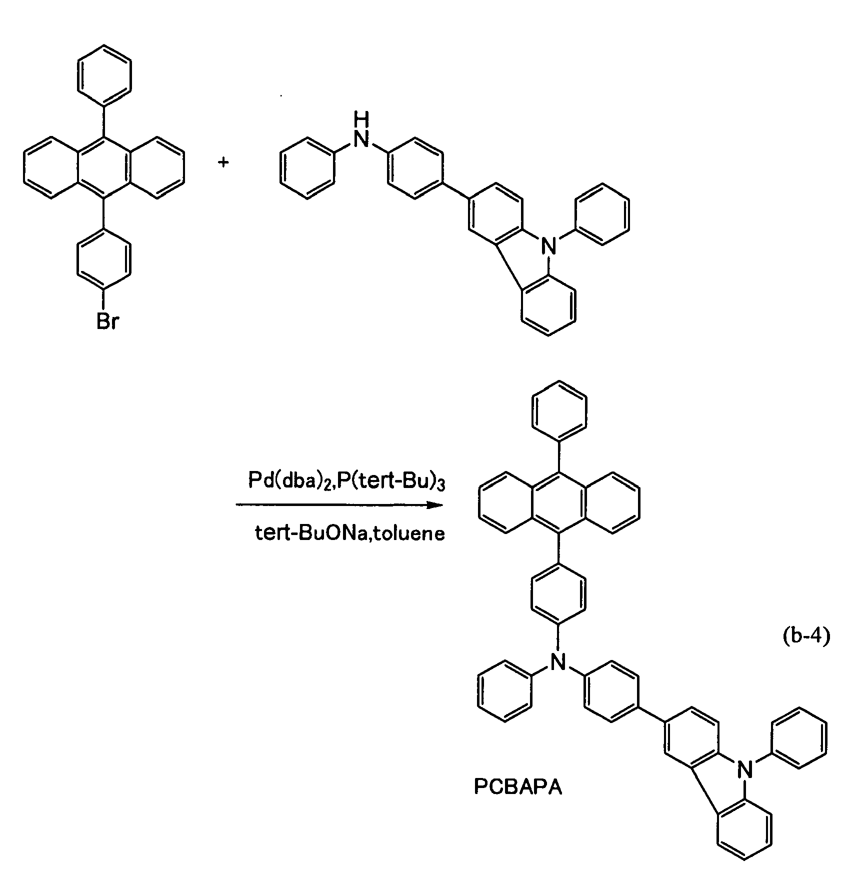

Note that the solid obtained in the above Step 4 was analyzed by ¹H NMR. The analysis data are shown below. From the analysis results, it can be seen that PCBAPA, which is the 9,10-diarylanthracene derivative represented by the above structural formula (13), was obtained.

¹H NMR (CDCl₃, 300MHz): δ = 7.09-7.14 (m, 1H), 7.28-7.72 (m, 33H), 7.88 (d, J = 8.4 Hz, 2H), 8.19 (d, J = 7.2 Hz, 1H), 8.37 (d, J = 1.5 Hz, 1H).

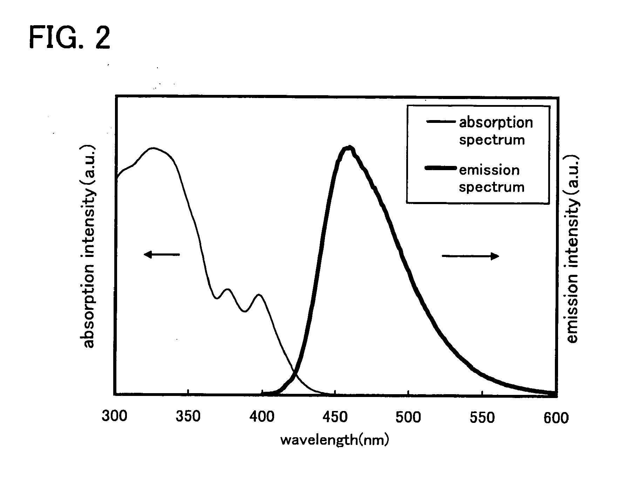

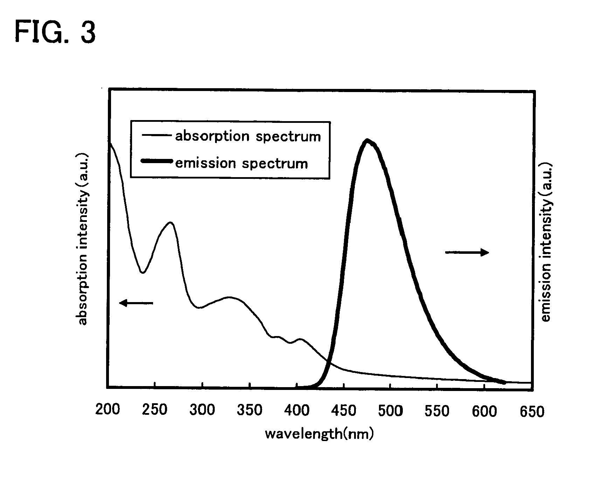

Next, the absorption spectrum of PCBAPA was measured with an ultraviolet-visible spectrophotometer (V-550, a product of JASCO Corporation) at room temperature using a toluene solution. The emission spectrum of PCBAPA was also measured with a fluorescence spectrophotometer (FS920, a product of Hamamatsu Photonics Corporation) at room temperature using a toluene solution. FIG 2 shows the measurement results. Further, measurements similar to that described above were conducted for a thin film of PCBAPA which was formed by an evaporation method. FIG. 3 shows the measurement results. In addition, the horizontal axis represents wavelength (nm), and the vertical axis represents absorption intensity (given unit) and emission intensity (given unit).

As can be seen from FIG. 2 and FIG. 3, light emission from PCBAPA in the toluene solution has a peak at 459 nm, and light emission from PCBAPA in the thin film has a peak at 473 nm. Hence, it is found that PCBAPA is an excellent light-emitting material that exhibits blue light emission with high color purity.

Thus, by using a synthesis method according to the present invention, a variety of materials having an anthracene skeleton can be simply and efficiently synthesized.

### [Example 4]

In Example 4, an example is described in which a 9,10-diarylanthracene derivative is synthesized by coupling of 9-iodo-10-phenylanthracene synthesized in Example 1 or Example 2 and aromatic hydrocarbon having a halogen group.

### «Synthesis Example 8: Synthesis of 9-Phenyl-9'-[4-(10-phenyl-9-anthryl)phenyl]-3,3'-bi(9H-carbazole) (abbreviation: PCCPA)»

In Synthesis Example 8, a method of synthesizing 9-phenyl-9'-[4-(10-phenyl-9-anthryl)phenyl]-3,3'-bi(9*H*-carbazole) (abbreviation: PCCPA) represented by a structural formula (14) below is described.

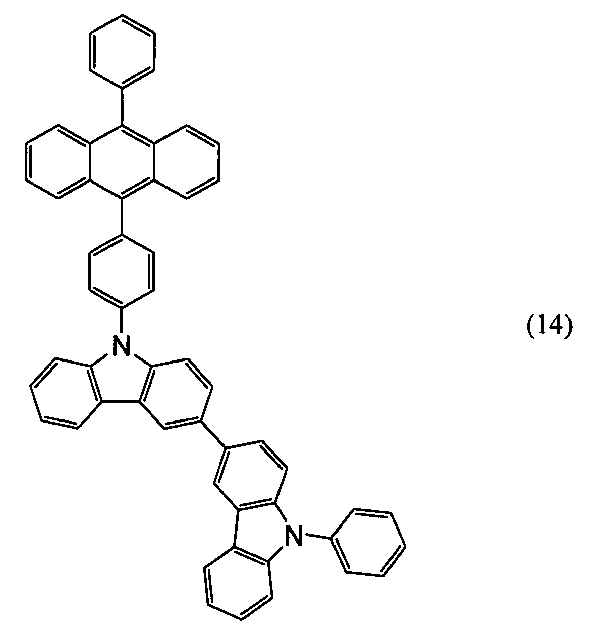

First, a method of synthesizing 9-phenyl-3,3'-bi(9*H*-carbazole) (abbreviation: PCC), which is coupled with 9-(4-bromophenyl)-10-phenylanthracene in order to synthesize the object, PCCPA, is described in Step 1.

### [Step 1: Synthesis of 9-Phenyl-3,3'-bi(9H-carbazole) (abbreviation: PCC)]

In a 200 mL three-necked flask were put 2.5 g (10 mmol) of 3-bromocarbazole, 2.9 g (10 mmol) of *N*-phenylcarbazole-3-boronic acid, and 152 mg (0.50 mmol) of tri(ortho-tolyl)phosphine. The atmosphere in the flask was replaced with nitrogen. To this mixture were added 50 mL of dimethoxyethanol (DME) and 10 mL of an aqueous potassium carbonate solution (2 mol/l). This mixture was degassed by being stirred while the pressure was reduced. After the mixture was degassed, to this mixture was added 50 mg (0.2 mmol) of palladium(II) acetate. The mixture was stirred under nitrogen stream at 80 °C for 3 hours. After the mixture was stirred, to this mixture was added about 50 mL of toluene, followed by stirring for about 30 minutes. This mixture was washed with water and a saturated saline solution in that order. After the mixture was washed, the organic layer was dried with magnesium sulfate. This mixture was gravity filtered, and the obtained filtrate was concentrated to give an oily substance. The obtained oily substance was dissolved in toluene, and this solution was filtered through Florisil (a product of Wako Pure Chemical Industries, Ltd., Catalog No. 540-00135), alumina, and Celite (a product of Wako Pure Chemical Industries, Ltd., Catalog No. 531-16855). The obtained filtrate was concentrated to give the object of the synthesis as 3.3 g of a white solid at a yield of 80 %. The synthesis scheme of Step 1 is illustrated in (c-1) below.

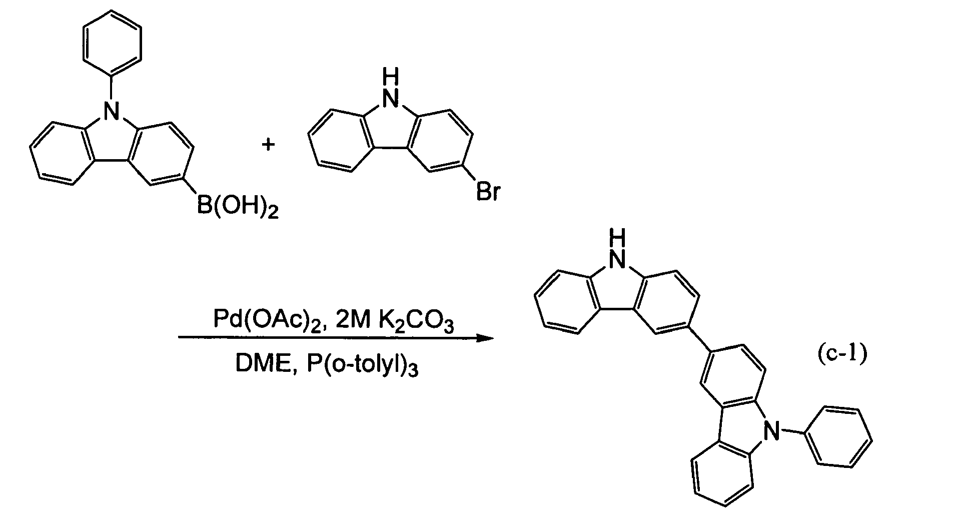

Next, a method of synthesizing PCCPA by coupling of 9-(4-bromophenyl)-10-phenylanthracene and PCC is described.

### [Step 2: Synthesis of PCCPA]

In a 100 mL three-necked flask were put 1.2 g (3.0 mmol) of 9-(4-bromophenyl)-10-phenylanthracene as synthesized in Step 1 of Synthesis Example 7 in Example 3, 1.2 g (3.0 mmol) of PCC, and 1.0 g (10 mmol) of sodium *tert*-butoxide. The atmosphere in the flask was replaced with nitrogen. To this mixture were added 20 mL of toluene and 0.1 mL of tri(tert-butyl)phosphine (a 10 wt% hexane solution). This mixture was degassed by being stirred while the pressure was reduced. After the mixture was degassed, to this mixture was added 96 mg (0.17 mmol) of bis(dibenzylideneacetone)palladium(0). This mixture was refluxed under nitrogen stream at 110 °C for 8 hours. After the mixture was refluxed, to this mixture was added about 50 mL of toluene, followed by stirring for about 30 minutes. This mixture was washed with water and a saturated saline solution in that order. After the mixture was washed, the organic layer was dried with magnesium sulfate. This mixture was gravity filtered, and the obtained filtrate was concentrated to give an oily substance. The obtained oily substance was purified by silica gel column chromatography (developing solvent, hexane:toluene = 1:1). The obtained light yellow solid was recrystallized with chloroform/hexane to give PCCPA, which was the object of the synthesis, as 1.2 g of a light-yellow powdered solid at a yield of 54 %. Then, 2.4 g of the obtained light-yellow powdered solid was sublimated and purified by train sublimation. For sublimation purification conditions, PCCPA was heated at 350 °C under a pressure of 8.7 Pa with argon gas at a flow rate of 3.0 mL/min. After the sublimation purification, PCCPA was obtained as 2.2 g of a light yellow solid at a yield of 94 %. In addition, the synthesis scheme of Step 2 is illustrated in (c-2) below.

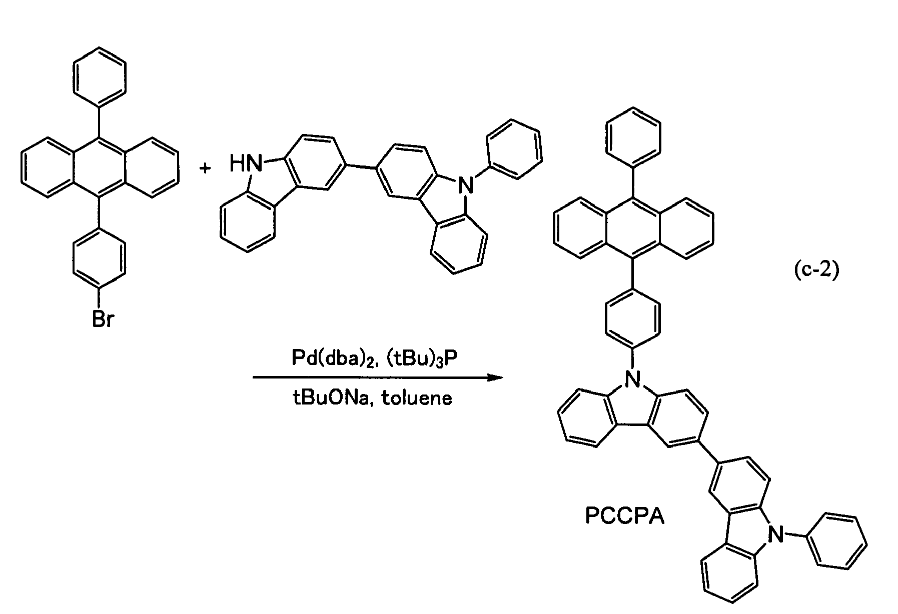

Note that the solid obtained in the above Step 2 was analyzed by ¹H NMR. The analysis data are shown below. From the analysis results, it can be seen that PCCPA, which is the 9,10-diarylanthracene derivative represented by the above structural formula (14), was obtained.

¹H NMR (CDCl₃, 300MHz): δ = 7.34-7.91 (m, 32H), 8.27 (d, J = 7.2 Hz, 1H), 8.31 (d, J = 7.5 Hz, 1H), 8.52 (dd, J₁ = 1.5 Hz, J₂= 5.4 Hz, 2H).

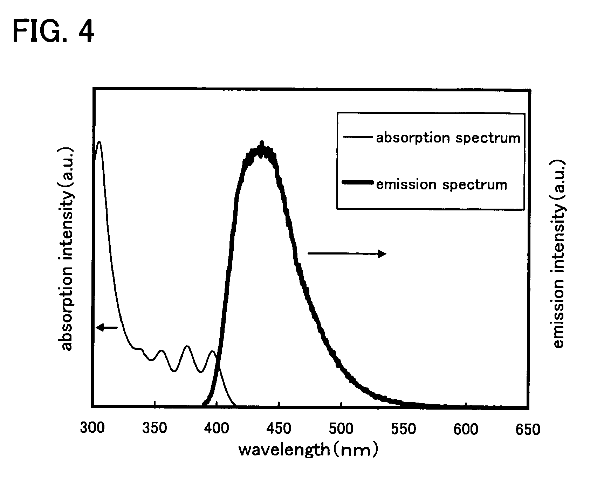

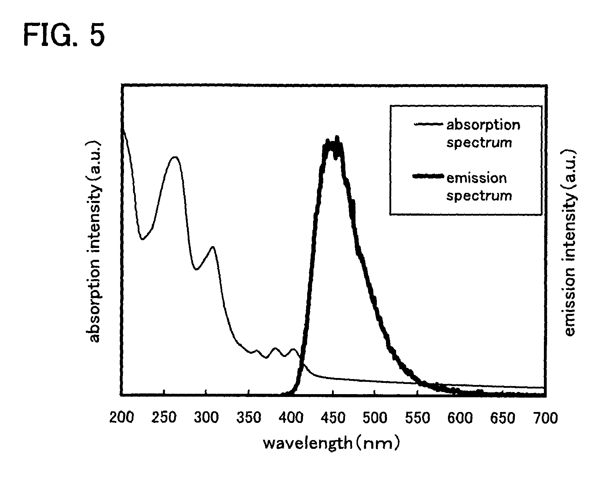

Next, the absorption spectrum of PCCPA was measured with an ultraviolet-visible spectrophotometer (V-550, a product of JASCO Corporation) at room temperature using a toluene solution. The emission spectrum of PCCPA was also measured with a fluorescence spectrophotometer (FS920, a product of Hamamatsu Photonics Corporation) at room temperature using a toluene solution. FIG. 4 shows the measurement results. Further, measurements similar to that described above were conducted for a thin film of PCCPA which was formed by an evaporation method. FIG 5 shows the measurement results. In addition, the horizontal axis represents wavelength, and the vertical axis represents molar absorption coefficient and emission intensity.

As can be seen from FIG. 4 and FIG. 5, light emission from PCCPA in the thin film has a peak at 454 nm, and light emission from PCCPA in the toluene solution has a peak at 436 nm. Hence, it is found that PCCPA is an excellent light-emitting material that exhibits blue light emission in particular.

Moreover, the redox characteristic of PCCAPA was measured by CV measurement. For the CV measurement, an electrochemical analyzer (ALS model 600a, a product of BAS Inc.) was used. Further, dimethylformamide (DMF) and tetra-*n*-butylammonium perchlorate (n-Bu₄NClO₄) were used as a solvent and a supporting electrolyte, respectively, and the concentration of tetra-*n*-butylammonium perchlorate was adjusted to be 10 mmol/L. Furthermore, PCCPA was dissolved in the electrolyte solution so that the concentration of PCCPA was adjusted to be 1 mmol/L. Further, a platinum electrode (a product of BAS Inc., PTE platinum electrode), a platinum electrode (a product of BAS Inc., Pt counter electrode for VC-3), and an Ag/Ag⁺ electrode (a product of BAS Inc., RE5 reference electrode for nonaqueous solvent) were used as a working electrode, an auxiliary electrode, and a reference electrode, respectively. Note that the scan rate was set to 0.1 V/s and the measurement was conducted for 100 cycles. From the results of this measurement, the oxidation potential of PCCPA was 0.47 V (vs. the Ag/Ag⁺ electrode). In addition, the reduction potential of PCCPA was -2.19 V (vs. the Ag/Ag⁺ electrode). Also from the results obtained after scanning for 100 cycles, a distinct redox peak of the CV curve was seen. Therefore, it is found that PCCPA which is the 9,10-diarylanthracene derivative synthesized in Example 4 is a stable substance having excellent reversibility of redoxes.

Thus, by using a synthesis method according to the present invention, a variety of materials having an anthracene skeleton can be simply and efficiently synthesized.

### [Example 5]

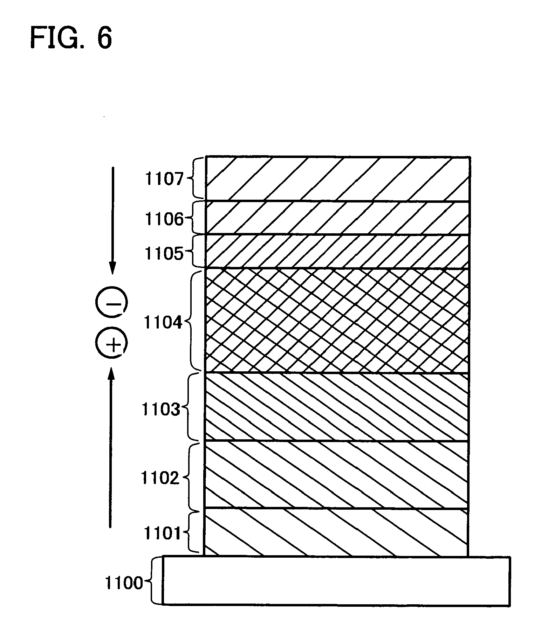

In Example 5, a light-emitting element fabricated using a light-emitting material (in Example 5, PCBAPA) that is synthesized using 9-aryl-10-iodoanthracene as a material is described with reference to FIG. 6. Chemical formulae of materials used in Example 5 are illustrated below.

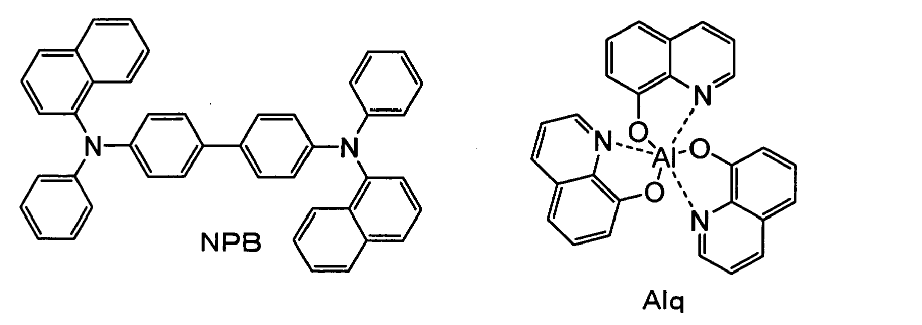

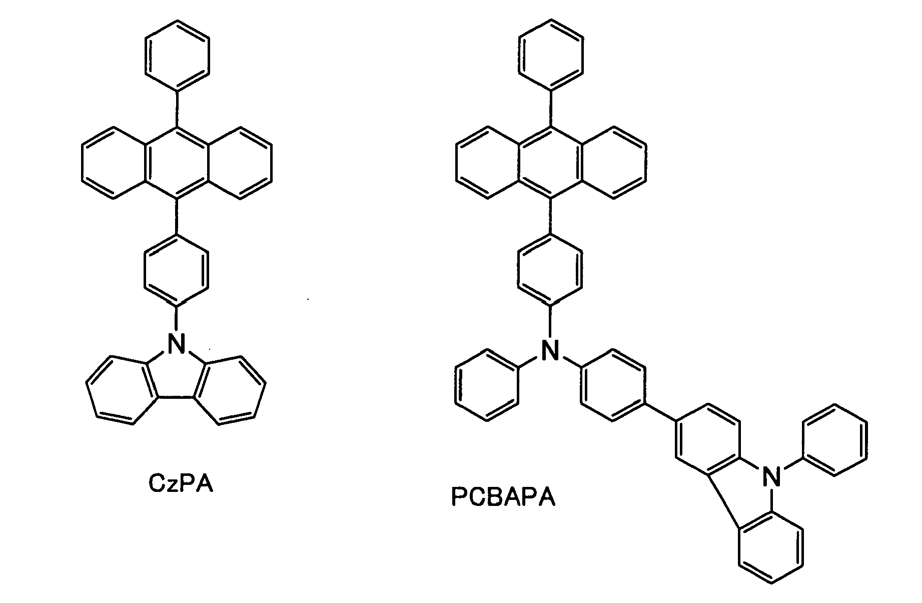

### (Fabrication of Light-Emitting Element)

First, a film of indium tin oxide containing silicon oxide was formed over a glass substrate 1100 by a sputtering method, whereby a first electrode 1101 was formed. Note that the thickness of the first electrode 1101 was set to 110 nm and the area of the electrode was set to 2 mm x 2 mm.

Next, the substrate provided with the first electrode was fixed to a substrate holder provided in a vacuum evaporation apparatus such that the side on which the first electrode was formed faced downward. After the pressure was reduced to about 10⁻⁴ Pa, NPB and molybdenum(VI) oxide were co-evaporated over the first electrode 1101, whereby a hole-injecting layer 1102 including a composite material of an organic compound and an inorganic compound was formed. The thickness of the hole-injecting layer 1102 was set to 50 nm and the weight ratio of NPB to molybdenum oxide was adjusted so as to be 4:1 (= NPB:molybdenum oxide). Note that a co-evaporation method refers to an evaporation method by which evaporation is concurrently conducted from a plurality of evaporation sources in one treatment chamber.

Next, a 10-nm-thick NPB film was formed over the hole-injecting layer 1102 by an evaporation method with resistance heating, whereby a hole-transporting layer 1103 was formed.

Furthermore, 9-[4-(10-phenyl-9-anthryl)phenyl]-9*H-*carbazole (abbreviation: CzPA) and PCBAPA were co-evaporated, whereby a 30-nm-thick light-emitting layer 1104 was formed over the hole-transporting layer 1103. Here, the weight ratio of CzPA to PCBAPA was adjusted to be 1:0.10 (= CzPA:PCBAPA).

Then, a 10-nm-thick film of tris(8-quinolinolato)aluminum(III) (abbreviation: Alq) was formed over the light-emitting layer 1104 by an evaporation method with resistance heating, whereby an electron-transporting layer 1105 was formed.

Furthermore, tris(8-quinolinolato)aluminum(III) (abbreviation: Alq) and lithium were co-evaporated over the electron-transporting layer 1105, whereby a 20-nm-thick electron-injecting layer 1106 was formed. Here, the weight ratio of Alq to lithium was adjusted to be 1:0.01 (= Alq:lithium).

Lastly, a 200-nm-thick aluminum film was formed over the electron-injecting layer 1106 by an evaporation method with resistance heating, whereby a second electrode 1107 was formed. Thus, the light-emitting element was fabricated.

### (Characteristics of Light-Emitting Element)

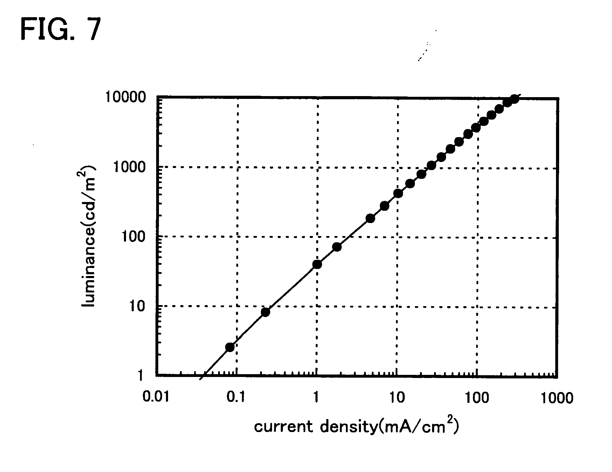

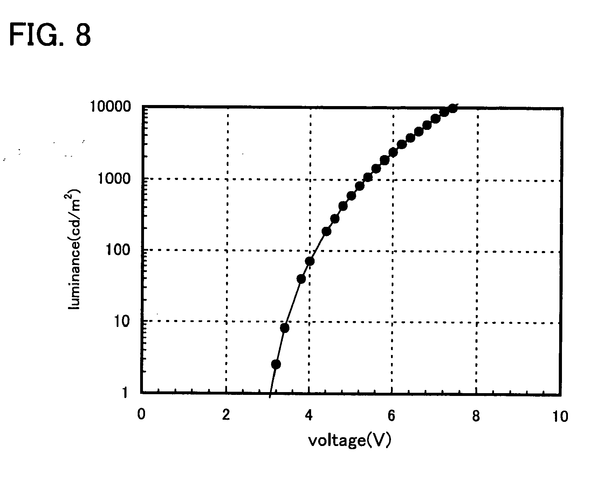

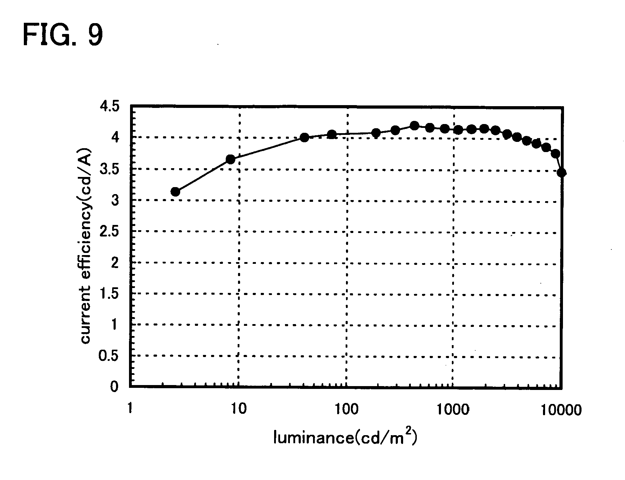

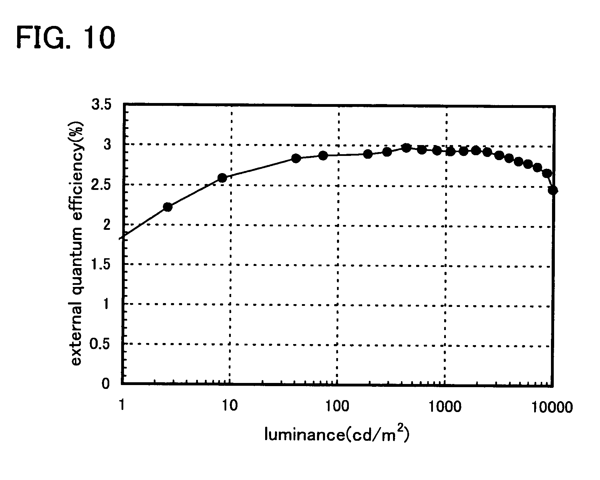

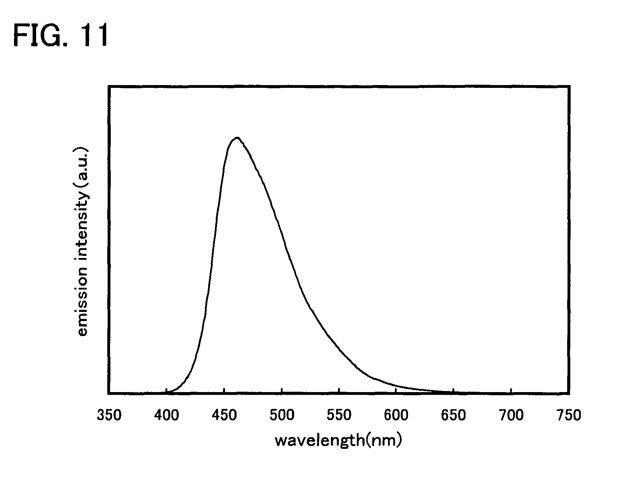

FIG. 7 shows the current density vs. luminance characteristic of the light-emitting element. FIG. 8 shows the voltage vs. luminance characteristic of the light-emitting element. FIG. 9 shows the luminance vs. current efficiency characteristic of the light-emitting element. FIG. 10 shows the luminance vs. external quantum efficiency of the light-emitting element. FIG. 11 shows the emission spectrum of the light-emitting element at a current of 1 mA. As can be seen from FIG 11, light emission from the light-emitting element was obtained from PCBAPA. The CIE chromaticity coordinates of the light-emitting element at a luminance of 820 cd/m² were (x, y) = (0.16, 0.19), and thus blue light emission with high color purity was obtained. Further, from FIG. 10, the external quantum efficiency of the light-emitting element at a luminance of 820 cd/m² was 2.9 %, and thus it is found that high external quantum efficiency was exhibited. Therefore, the light-emitting element had high emission efficiency. Further, from FIG. 9, the current efficiency of the light-emitting element at a luminance of 820 cd/m² was 4.2 cd/A, and thus it is found that the light-emitting element had high luminous efficiency. Furthermore, from FIG 8, the driving voltage of the light-emitting element at a luminance of 820 cd/m² was 5.2 V, and thus it is found that a voltage required to obtain a certain luminance was low and power consumption was also low.

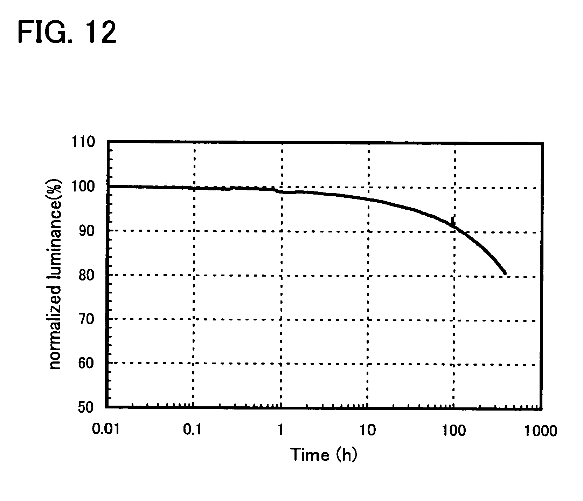

Note that the light-emitting element of Example 5 was driven at a constant current with the initial luminance thereof set to 1000 cd/m², whereby a luminance degradation curve as shown in FIG 12 was obtained. In FIG 12, the horizontal axis represents time and the vertical axis represents relative luminance (%) on condition that the initial luminance was set to 100. The light-emitting element fabricated in Example 5 kept 81 % of the initial luminance after driving for 380 hours.
The present application is based on Japanese Patent Application serial No. 2008-077893 filed with Japan Patent Office on March 25, 2008, the entire contents of which are hereby incorporated by reference.

## Claims

1. A method of synthesizing 9-aryl-10-iodoanthracene, wherein iodine is introduced into a 10-position of 9-arylanthracene by mixing an iodinating agent that is a substance having an amide bond in which iodine is directly bonded to nitrogen of the amide bond, an acid, and 9-arylanthracene.

2. The method of synthesizing 9-aryl-10-iodoanthracene, according to claim 1, wherein the iodinating agent is a substance having a diacylamide structure.

3. The method of synthesizing 9-aryl-10-iodoanthracene, according to claim 1, wherein the iodinating agent is a substance having an imide structure.

4. The method of synthesizing 9-aryl-10-iodoanthracene, according to claim 1, wherein the acid is a Bronsted acid.

5. The method of synthesizing 9-aryl-10-iodoanthracene, according to claim 1, wherein the acid is any of acetic acid, sulfuric acid, or trifluoroacetic acid.

6. A method of synthesizing 9-iodo-10-phenylanthracene, wherein the 9-arylanthracene according to claim 1 is 9-phenylanthracene.

7. A method of synthesizing a light-emitting material, wherein the 9-aryl-10-iodoanthracene synthesized by the method according to claim 1 and aromatic hydrocarbon including halogen are coupled.

8. A method of synthesizing 9-aryl-10-iodoanthracene, wherein iodine is introduced into a 10-position of 9-arylanthracene by mixing an iodinating agent that is a substance having an amide bond in which iodine is directly bonded to nitrogen of the amide bond, an acid, a solvent, and 9-arylanthracene.

9. The method of synthesizing 9-aryl-10-iodoanthracene, according to claim 8, wherein the iodinating agent is a substance having a diacylamide structure.

10. The method of synthesizing 9-aryl-10-iodoanthracene, according to claim 8, wherein the iodinating agent is a substance having an imide structure.

11. The method of synthesizing 9-aryl-10-iodoanthracene, according to claim 8, wherein the acid is a Bronsted acid.

12. The method of synthesizing 9-aryl-10-iodoanthracene, according to claim 8, wherein the acid is any of acetic acid, sulfuric acid, or trifluoroacetic acid.

13. A method of synthesizing 9-iodo-10-phenylanthracene, wherein the 9-arylanthracene according to claim 8 is 9-phenylanthracene.

14. A method of synthesizing a light-emitting material, wherein the 9-aryl-10-iodoanthracene synthesized by the method according to claim 8 and aromatic hydrocarbon including halogen are coupled.
